# EUROPEAN PATENT APPLICATION

(11) **EP 4 218 549 A1**
(43) Date of publication of application: **02.08.2023**
(21) Application number: 23153565.9
(22) Date of filing: 26.01.2023
(51) Int. Cl.: A61B 5/00

(54) **WIRELESSLY POWERED SENSORS FOR ORTHOPEDIC IMPLANTS**

(30) Priority: 26.01.2022 US 202263303200 P
(71) Applicant: Orthosoft, Inc., Montreal, Québec H3C 2N6 (CA)
(72) Inventor: LEVEILLE, Catherine, Longueuil, J4H2B8 (CA); CASAUBON, Jerome, Montreal, H3C 2N6 (CA)
(74) Representative: Mays, Julie

(57) **Abstract**

A surgical sensor system for collecting internal patient data comprises a prosthetic implant comprising a housing, a sensor disposed within the housing and an internal power device connected to the sensor; and an external interrogation device comprising a wireless power signal generator for activating with the internal power device of the prosthetic implant. A method of remotely interacting with a sensor device implanted in anatomy with an orthopedic device comprises generating a wireless powering signal, activating the sensor device with the wireless power signal, collecting sensor data from the sensor device, and wirelessly communicating the sensor data from the sensor device using a low-power wireless signal. A method comprises generating wireless powering signals within an operating room using an interrogation device, activating electronics within a sensor-enabled orthopedic device with the signals, collecting data from the electronics, and wirelessly communicating data from the electronics to the interrogation device.

## Description

### TECHNICAL FIELD

The present disclosure is generally directed to, but not by way of limitation, systems, devices and methods incorporating sensors for use in performing, monitoring and evaluating medical procedures, such as arthroplasty procedures.

### BACKGROUND

Arthroplasty procedures involve the implantation of medical devices, e.g., orthopedic implants, into anatomy of a patient. Typically, once the medical device is implanted into the patient, or even while it is being implanted, it is difficult to obtain feedback regarding the effectiveness of the implant or the implant procedure. Attempts have been made to obtain data from orthopedic implants using sensors.

Pub. No. US 2018/0125365 to Hunter et al. is titled "Devices, Systems and Methods for Using and Monitoring Medical Devices."

Pub. No. US 2019/0350518 to Bailey et al. is titled "Implantable Reporting Processor for an Implant."

Pat. No. US 10,492,686 to Hunter et al. is titled "Devices, Systems and Methods for Using and Monitoring Medical Devices."

### OVERVIEW

The present inventors have recognized, among other things, that problems to be solved with conventional sensor systems used in conjunction with implantable medical devices, such as orthopedic implants, involve the necessity to provide power to sensors and electronics used in conjunction with the implanted medical device. Previously designed orthopedic implants have relied on batteries to be implanted along with the prosthetic component. Use of batteries can impact both the physical design of the prosthetic component and the performance of the sensors. For example, the prosthetic component must be large enough to accommodate the battery and sensor. Increase in size of the orthopedic implant can result in removal of excess bone from the patient to accommodate the larger implant with the battery or can result in constraints being placed on the form factor of the implantable medical device. Furthermore, because the battery can have a finite life or can only be recharged in certain circumstances, powering of the sensors is typically conducted on an intermittent basis, which can result in infrequent data collection and incomplete sensor data.

The present subject matter can provide a solution to these and other problems, such as by providing sensor-enabled orthopedic implants that can be provided with or generate power wirelessly, such as through inductive charging with a mobile computing device or via radio frequency (RF) signals.

Furthermore, the present inventors have recognized that recharging battery-powered medical devices can be burdensome to the patient by requiring the patient to wear recharging equipment at inconvenient times or limiting the time a patient can be away from an external power source.

The present subject matter can provide a solution to these and other problems, such as by providing RF energy harvesters that can be powered in the home by RF generators or that can be powered outside the home by stray RF energy in the environment.
1) The present subject matter includes the use of sensor-enabled implantable orthopedic devices that can be wirelessly charged, such as via induction or RF energy, by mobile computing devices during typical usage or carrying of the mobile computing device by the patient, thereby reducing the burden on the patient of having to connect to battery chargers.
2) The present subject matter includes the use of sensor-enabled implantable orthopedic devices that can be charged by radio frequency energy, such as by use of an energy harvesting device, thereby enabling the orthopedic implant to be charged in the home via a home base station that can generate RF signals or outside the home by stray radio frequency energy.
3) The present subject matter includes the use of sensor-enabled implantable orthopedic devices that can be charged by radio frequency energy, such as by use of an energy harvesting device, thereby enabling the orthopedic implant to be charged via a portable device, such as a mobile phone, dongle or wearable module, that can generate RF signals or outside the home by stray radio frequency energy.

In an example, a surgical sensor system for collecting internal patient data can comprise a prosthetic implant comprising a housing, a sensor disposed within the housing and an internal power device connected to the sensor; and an external interrogation device comprising a wireless power signal generator for activating with the internal power device of the prosthetic implant.

In an additional example, a medical implant sensor interrogation device can comprise a communication interface configured to communicate with the Internet via a wireless signal, a radio frequency signal generator configured to activate a radio frequency energy harvester, and a sensor communication device configured to generate a low-power, human anatomy compatible communication signal.

In another example, a method of remotely interacting with a sensor device implanted in anatomy with an orthopedic device can comprise generating a wireless powering signal, activating the sensor device with the wireless power signal, collecting sensor data from the sensor device, and wirelessly communicating the sensor data from the sensor device using a low-power wireless signal.

In another example, a method of intraoperatively receiving sensor data from an implantable sensor-enabled orthopedic device can comprise generating a wireless powering signal within an operating room using an interrogation device, activating electronics within the orthopedic device with the wireless power signal, collecting sensor data from the electronics, and wirelessly communicating sensor data from the electronics to the interrogation device.

### BRIEF DESCRIPTION OF THE DRAWINGS

FIG. 1 is an exploded view of a total knee arthroplasty system comprising a tibial component, a tibial bearing and a femoral component.
FIG. 2 is an exploded view of the tibial bearing of FIG. 1 showing an electronics component located between upper and lower housing components.
FIG. 3 is a top view of the tibial bearing of FIGS. 1 and 2 showing various sensors in phantom below the top housing component.
FIG. 4 is a schematic view of a display screen showing a representation of output of the pressure sensors of FIG. 3.
FIG. 5A is a front view of a patient having a sensor-enabled total hip arthroplasty system and wearing a mobile computing device.
FIG. 5B is a schematic view of the implanted total hip arthroplasty system of FIG. 5A communicating with and receiving power from the mobile computing device.
FIG. 6 is a schematic illustration of the tibial bearing of FIGS. 1 - 3 communicating with and receiving power from a home base station, which is in communication with a wireless router.
FIG. 7 is a schematic illustration of the tibial bearing of FIGS. 1 - 3 communicating with and receiving power from a dongle, which is in communication with a wireless router.
FIG. 8 is a schematic illustration of the tibial bearing of FIGS. 1-3 communicating with and receiving power from a mobile computing device that is cloud-connected.
FIG. 9 is a schematic illustration of tibial bearing of FIGS. 1-3 communicating with and receiving power from a wearable module attached to a joint brace that is in communication with a wireless router.
FIG. 10 is a schematic view of the electronics component of FIGS. 2 and 3 showing various sensors, communication devices and energy harvesting devices.

### DETAILED DESCRIPTION

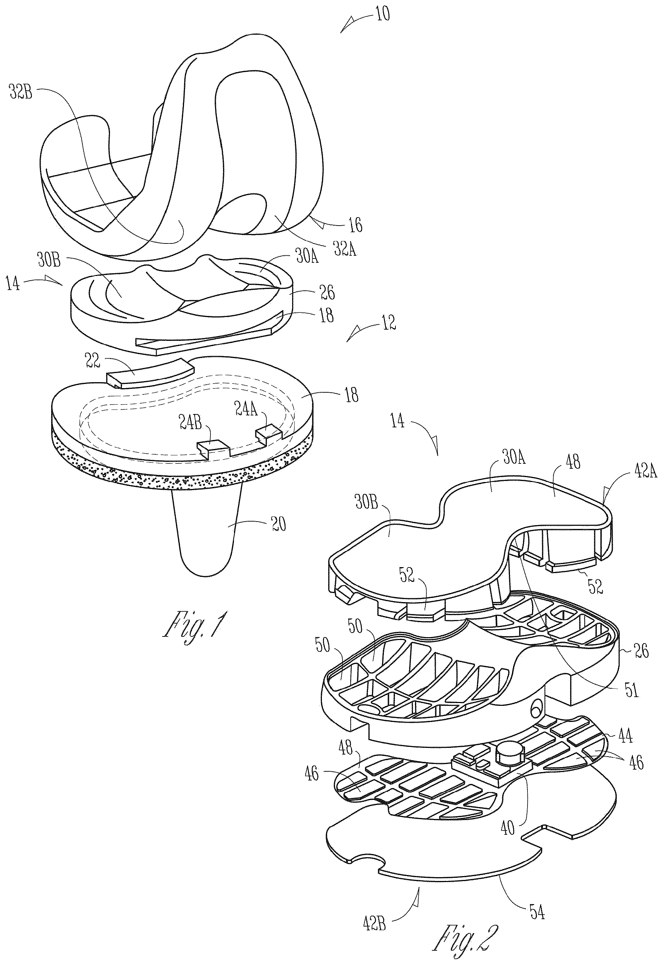

FIG. 1 is a perspective view of total knee prosthesis 10 comprising tibial component 12, tibial bearing 14 and femoral component 16.

Tibial component 12 can be connected to a proximal end of a tibia after the tibia has been resected. Tibial component 12 can comprise baseplate 18 and stem 20. Baseplate 18 can comprise features, such as posterior lip 22 and anterior flanges 24A and 24B, for coupling tibial bearing 14 to tibial component 12. Baseplate 18 can be configured to rest flush on top of the resected tibia surface while stem 20 extends into the intramedullary canal of the tibia. Tibial component 12 can be affixed to the tibia bone by any suitable means, such as bone cement. Tibial component 12 can provide a firm platform for tibial bearing 14 that is aligned with the axis of the tibia for kinematic alignment with the femur.

Tibial bearing 14 can comprise housing 26 having interlocking feature 28 for receiving anterior flanges 24A and 24B and a posterior feature (not visible) for receiving posterior lip 22. As such, tibial bearing 14 can be immobilized relative to tibial component 12. Articular surfaces 30A and 30B can be configured to receive mating components of femoral component 16. Articular surfaces 30A and 30B can comprise depressions or divots that allow for articulation, e.g., rolling or sliding, of artificial condyle components of femoral component 16.

Femoral component 16 can be rigidly connected to a distal end of a femur after the femur has been resected. Femoral component 16 can comprise first condylar portion 32A and second condylar portion 32B. Condylar portions 32A and 32B can comprise articulating surfaces configured to engage tibial bearing 14. Condylar portions 32A and 32B can comprise curved or rounded surfaces that rotate in the depressions or divots of articular surfaces 30A and 30B, respectively, of tibial bearing 14. Femoral component 16 can be affixed to the femur bone by any suitable means, such as bone cement. Femoral component 16 can be aligned with the axis of the tibia for kinematic alignment with the tibia.

Examples of total knee prosthesis 10 are described in Pub. No. US 2010/0100191 to May et al. titled "TIBIAL TRAY HAVING A REINFORCING MEMBER," which is incorporated herein by this reference.

Tibial bearing 14 can be made from any suitable material, such as ultra-high molecular weight polyethylene (UHMWP) or stainless steel. As discussed with reference to FIG. 2, housing 26 of tibial bearing 14 can comprise housing components that can include internal spaces for receiving electronics module 40 (FIG. 2), including sensors such as pressure sensors, communication devices and energy generating components. Tibial bearing 14 can come in different shapes and sizes to allow the surgeon to select a tibial bearing 14 that will mate with femoral component 16 to provide suitable kinematic alignment, thereby providing the patient with a comfortable and realistic feeling artificial knee joint.

In order for the surgeon to adequately evaluate the kinematic alignment of the artificial knee joint, such as with different sized tibial bearings 14, it can be desirable to obtain data from total knee prosthesis 10. As such, electronics module 40 (FIG. 2) of tibial bearing 14, or another component of total knee prosthesis 10, can be provided with various sensors, including pressure sensors, to provide the surgeon with an indication of the force being generated between articular surfaces 30A and 30B and condylar portions 32A and 32B, respectively. Intraoperative feedback, e.g., pressure data, can be useful for the surgeon in determining kinematic alignment. Postoperative feedback, e.g., pressure data, impact data, etc., can be useful for the surgeon in evaluating performance of total knee prosthesis 10.

Furthermore, electronics module 40 (FIG. 2) of tibial bearing 14, or another component of total knee prosthesis 10, can be provided with energy harvesting devices to allow the sensors to be conveniently charged with minimal patient intervention and low-power communication devices that can transmit the sensor data outside total knee prosthesis 10 with low power drain. In examples, the electronics can include energy harvesting devices that are described in Pat. No. US 9,021,277 to Shearer et al. (Powercast) titled "Powering Devices Using RF Energy Harvesting," which is incorporated herein by this reference. In examples, the electronics can communicate over extremely low power wireless communication networks designed for use in the human body, as described in IEEE 802.15.6-2012 - IEEE Standard for Local and metropolitan area networks - Part 15.6: Wireless Body Area Networks. In additional examples, MedRadio communication protocols, such as Medical Implant Communication Service (MICS) and Medical Body Area Networks (MBANs) can be used.

The energy harvesting devices can comprise devices for converting radio frequency (RF) signals into power. Thus, the implants can be powered in the home with RF generators integrated into data interrogation devise as well as with ambient or stray RF signals when outside the home. The low-power communication signals can facilitate RF energy harvesting, particularly with stray RF signals, by not requiring the energy harvesting devices to be exposed to strong RF fields. As such the systems, devices and methods described herein can allow a patient to go about their life with minimal interruptions from operating the electronics of an implanted medical device. That is, a home base station or dongle can be used to communicate with and provide power to fully operate the implanted medical device when the user is at home or another environment where they have prolonged exposure. However, the implanted medical device can continue to operate while away from the home base station or dongle by taking advantage of ambient, environmental or stray RF fields.

Although the present application has thus far been described with reference to a total knee arthroplasty system, the present disclosure can be used with other orthopedic implant systems, such as hip, shoulder, ankle and others. For example, various prosthetic acetabular cups, glenoid devices, femoral heads, humeral heads and the like can include sensors for measuring various parameters, such as pressure and temperature, as well as low-power communication devices, wireless recharging devices and energy harvesting devices, as discussed herein.

FIG. 2 is an exploded view of tibial bearing 14 of FIG. 1 showing electronics module 40 located between superior housing component 42A and inferior housing component 42B. Examples of tibial bearing 14 are described in Pub. No. US 2013/0261758 to Claypool et al. titled "TIBIAL PROSTHESIS SYSTEMS, KITS, AND METHODS," which is incorporated herein by this reference. Examples of tibial bearing 14 are described in Pat. No. US 10,531,826 to Wasielewski et al. titled "Smart Joint Implant Sensors," which is incorporated herein by this reference. However, other tibial bearings can be used.

Tibial bearing 14 can comprise housing 26, superior housing component 42A and inferior housing component 42B and sensor plate 44. Sensor plate 44 can include one or more sensors 46 and electronics module 40, which can be disposed on superior surface 48. Tibial bearing 14 can be adapted for coupling to tibial component 12 (FIG. 1).

Housing 26 can comprise a plurality of apertures or openings 50 that can be sized and/or shaped to correspond to the plurality of sensors 46 on sensor plate 44. Superior housing component 42A can comprise superior surface 48 having articular surfaces 30A and 30B and non-articulating side 51 having extensions 52. Extensions 52 can be configured to extend through openings 50 to contact sensors 46. Inferior housing component 42B can comprise a plate configured to support sensor plate 44 and can form a bottom non-articulating side 54 of tibial bearing 14.

Sensors 46 can comprise pressure sensors. Sensors 46 can comprise any suitable force or pressure sensors or readers, such as, but not limited to, piezoelectric sensors, force sensing resistors, force gauges, strain gauges, load cells, potentiometers, barometers, or the like. Example force sensors include force sensing resistor or capacitive flex circuits, piezoelectric film, piezoelectric elements, piezoresistive and piezoelectric polymers, metal foil strain gages, semiconductor strain gages, piezoresistive and capacitive pressure sensors, interferometric optical sensors, path displacement optical sensors, optical fiber force sensors, and other suitable sensing technologies.

Sensors 46 can additionally comprise other types of sensors, such as temperature sensors, accelerometers, gyroscopes, multi-axis sensors, inertial sensors, moisture sensors and others.

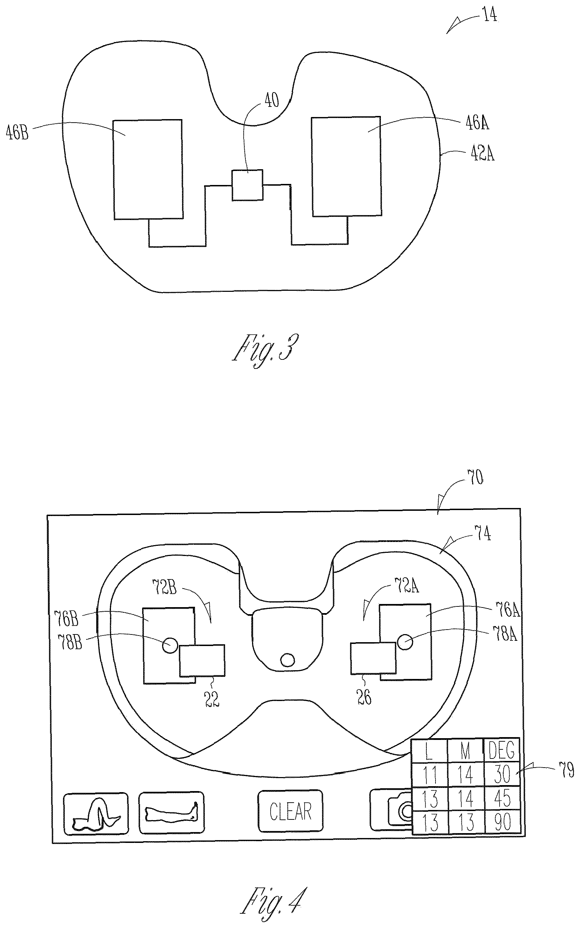

Sensors 46 can occupy a substantial portion of superior surface 48 of sensor plate 44 such that sensors 46 align with a substantial portion of the superior surface 48 of tibial bearing 14. Sensors 46 can be arranged in an array of columns and rows, as shown in FIG. 2, or can have other arrangements, as shown in FIG. 3. Sensor plate 44 can have a medial side M, a lateral side L, an anterior side A, and a posterior side P, all of which can similarly apply to other components of total knee prosthesis 10. By configuring sensors 46 to be generally spaced over a substantially portion of a surface that is parallel to, and aligned with, superior surface 48, sensors 46 can facilitate precise sensing on both the medial M and lateral L sides and/or anterior A and posterior P sides. Similarly, sensors 46 can facilitate deep posterior sensing, which can be beneficial when determining high-flex balance and roll-back, and/or to predict wear, for example. Output of data gathered with sensors 46 is described with reference to FIGS. 3 and 4.

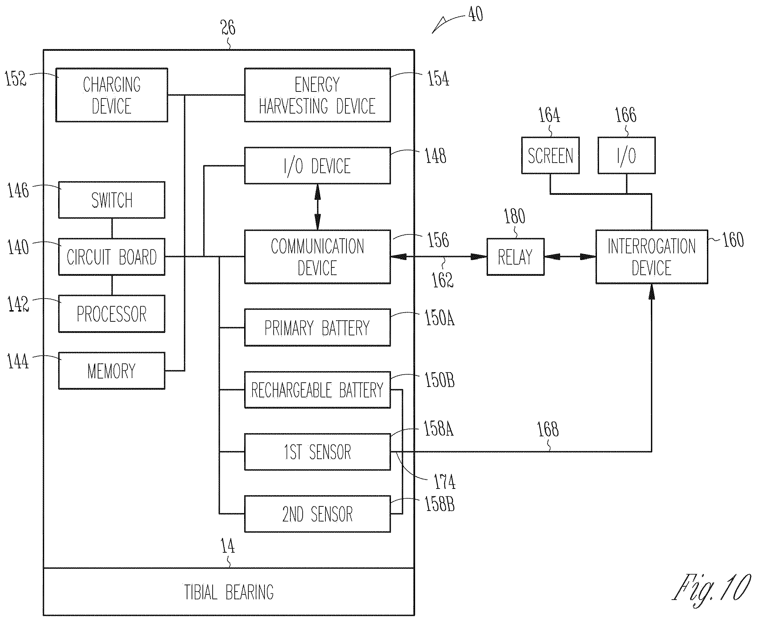

Electronics module 40 can comprise components for receiving, processing and communicating signals obtained from sensors 46. Electronics module 40 can comprise wireless communication circuitry, battery charging circuitry, energy harvesting circuitry and others. Details of examples of electronics module 40 are described with reference to FIG. 10.

FIG. 3 is a top view of tibial bearing 14 of FIGS. 1 and 2 showing pressure sensor arrays 46A and 46B in phantom below superior housing component 42A. Pressure sensor arrays 46A and 46B can be positioned at medial and lateral sides of superior housing component 42A to receive corresponding pressures of medial and lateral condyle portions 32A and 32B of femoral component 16 (FIG. 1). In examples, pressure sensor arrays 46A and 46B can comprise three or more sensors configured to triangulate a central sensor reading, as well as posterior, anterior, medial and lateral pressure readings. Output of pressure sensor arrays 46A and 46B can be communicated to electronics module 40 via appropriate connections, such as wires, traces and the like.

FIG. 4 is a schematic view of display screen 70 showing representations 72A and 72B of output of pressure sensors 46A and 46B of FIG. 3 inside an outline of tibial bearing 14 indicated as bearing image 74. Display screen 70 can additionally comprise sensor images 76A and 76B, and center points 78A and 78B.

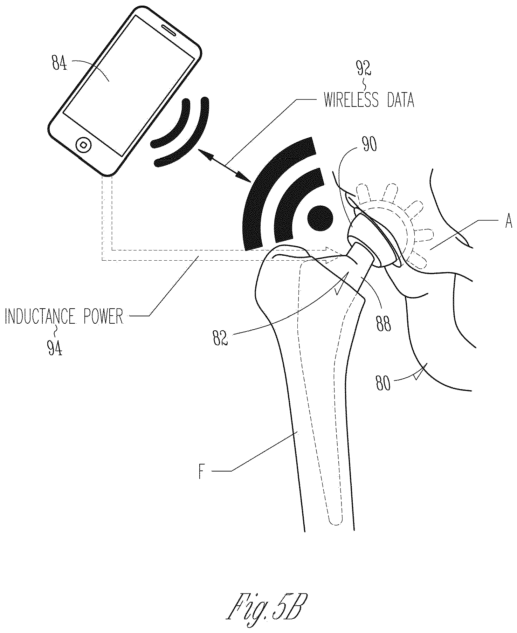

Display screen 70 can provide visual indicia of data collected by sensor arrays 46A and 46B as well as other sensors and other information. In the illustrated example, representations 72A and 72B indicated pressures imparted to superior housing component 42A at center points 78A and 78B. Center points 78A and 78B can represent the distal tips of condyle portions 32A and 32B and/or the centers of articular surfaces 30A and 30B, respectively. Pressures at other locations on superior housing component 42A can additionally be shown. Information from other types of pressures can also be shown with other indicia 79. Display screen 70 can be generated by various computing devices, such as mobile computing device 84 (FIG. 5B) and interrogation device 160 (FIG. 10). Display screen 70 can additionally be viewed on other computing devices such as tablets, notebook computers and other devices that can receive data from tibial bearing 14 via the cloud or internet or other wireless and wired connections. Data from sensors 46 (FIG. 2), sensor arrays 46A and 46B (FIG. 3) and other sensors can be collected by various interrogation devices, such as mobile phones, base stations, dongles and the like.

As discussed herein, data from sensors 46 (FIG. 2), sensor arrays 46A and 46B (FIG. 3) and other sensors can be used intraoperatively, e.g., during the implantation procedure for total knee prosthesis 10 (FIG. 1), so that the surgeon performing the implantation procedure, or other personnel, can evaluate the implantation of total knee prosthesis 10, such as the kinematic alignment of the device and the like. Additionally, data from sensors 46 (FIG. 2), sensor arrays 46A and 46B (FIG. 3) and other sensors can be used postoperatively, e.g., after the implantation procedure for total knee prosthesis 10 (FIG. 1), so that the surgeon, the patient, rehabilitation therapists and others can evaluate the patient and total knee prosthesis 10.

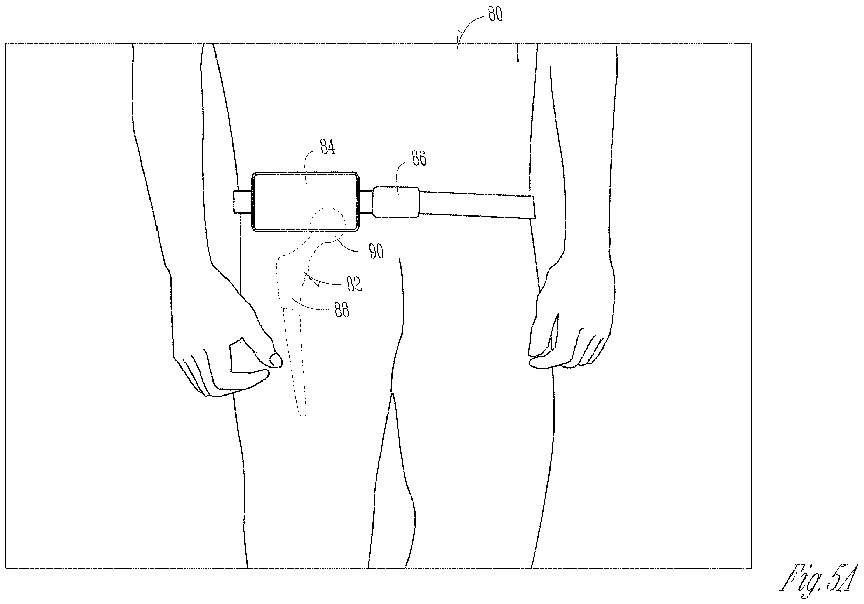

FIG. 5A is a front view of patient 80 having total hip arthroplasty system 82 wearing mobile computing device 84 attached to belt 86. FIG. 5B is a schematic view of implanted total hip arthroplasty system 82 of FIG. 5A communicating with mobile computing device 84. FIGS. 5A and 5B are discussed concurrently.

Total hip arthroplasty system 82 is shown in phantom in FIG. 5A to show the locations of total hip arthroplasty system 82 and mobile computing device 84 relative to patient 80. Patient 80 can have total hip arthroplasty system 82 implanted into femur F (FIG. 5B) and acetabulum A (FIG. 5B) of a skeletal system. In particular, femoral stem 88 can be implanted into femur F and acetabular cup 90 can be implanted into acetabulum A of patient 80, as is known in the art.

One or both of femoral stem 88 and acetabular cup 90 can include an electronics component, similar to electronics module 40 and associated sensors of FIG. 2, to allow for wireless communication with mobile computing device 84 via signal 92. As such, data from the operation of total hip arthroplasty system 82 can be generated and obtained in order to evaluate the performance of total hip arthroplasty system 82 and the health and wellbeing of patient 80. For example, the pressure of a femoral head on femoral stem 88 against acetabular cup 90 can be measured and monitored. In order for the sensors to operate, power can be provided to total hip arthroplasty system 82 from mobile computing device 84.

In examples, mobile computing device 84 can comprise a mobile or cellular phone. Examples of mobile computing device 84 are described in Pat. No. US 9,986,086 to Kim (Samsung) titled "Mobile Terminal and Method of Operating the Same," which is incorporated herein by this reference.

In examples, mobile computing device 84 can provide power to total hip arthroplasty system 82 from power stored in one or more batteries within mobile computing device 84. As such, mobile computing device 84 can generate wireless power-sharing signal 94. In examples, the wireless power-sharing signal can comprise an electromagnetic field or an inductance field. Examples of wireless power-sharing signals suitable that can comprise signal 94 are described in Pat. No. US 9,860,358 to Park et al. (Samsung) titled "Apparatus and Method for Sharing Energy in Wireless Device," which is incorporated herein by this reference. Examples of wireless power-sharing signals that can comprise signal 94 are described in Pat. No. US 9,107,579 to Greene (Powercast) titled "Systems, Methods and Apparatus for Powering Devices Using RF Energy From a Mobile Transmitter," which is incorporated herein by this reference.

As can be seen in FIG. 5A, mobile computing device 84 can be worn by patient 80 in a manner consistent with every-day usage. For example, belt 86 can be connected to a pouch or case in which mobile computing device 84 can be inserted, thereby positioning mobile computing device 84 in close proximity to total hip arthroplasty system 82. Thus, total hip arthroplasty system 82 can be positioned within range of wireless power-sharing signal 94. As such, mobile computing device 84 can act to power electronics within total hip arthroplasty system 82 while patient 80 is away from home and partaking in activities that generate useful data while using mobile computing device 84 in a general manner, thereby reducing the burden of patient 80 to have to take particular steps to charge total hip arthroplasty system 82.

Additionally, mobile computing device 84 can be held in close proximity to total hip arthroplasty system 82 via positioning in a pocket on pants of patient 80. In additional examples, mobile computing device 84 can be used in conjunction with total knee arthroplasty systems, such as by using appropriate pouches or holsters that can attach to belt 86 or by positioning in thigh pockets or cargo pockets.

Thus, via the use of mobile computing device 84 and a user-wearable positioning device, such as belt 86, a pouch, belt clip, sling or holster, mobile computing device 84 can be used in an every-day manner to interact with total hip arthroplasty system 82.

In additional examples, total hip arthroplasty system 82 can be charged using other methods. For example, mobile computing device 84 can generate a radio frequency (RF) field that can be used to power an energy harvester in total hip arthroplasty system 82. Thus, when mobile computing device 84 is located away from patient 80, total hip arthroplasty system 82 can be charged using RF energy harvesting techniques described herein.

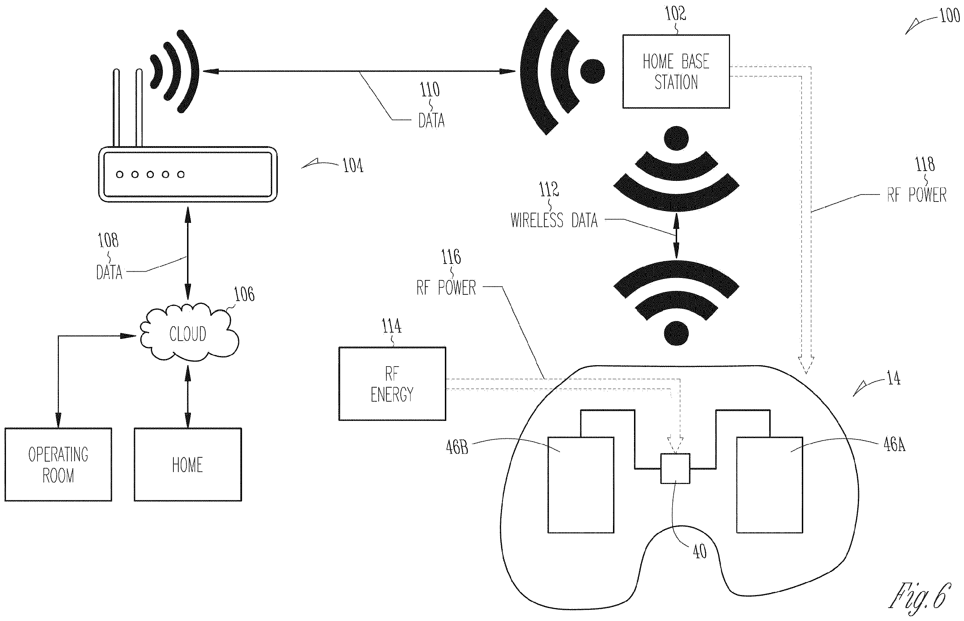

FIG. 6 is a schematic illustration of implant monitoring system 100 comprising tibial bearing 14 of FIGS. 1-3 communicating with base station 102, which is in communication with wireless router 104.

Wireless router 104 can be configured to communicate with the internet or the cloud 106 via signal 108. Thus, wireless router 104 can be configured to transmit data to and receive data from the cloud 106. Wireless router 104 can additionally operate with various wired connections. Wireless router 104 can additionally be configured to transmit data to and receive data from base station 102.

Base station 102 can be setup in the home of a patient or in another location where the patient spends time, such as a workplace or vacation destination. Base station 102 can be configured to connect to the cloud 106 via wireless router 104 or any other internet connection using communication signal 110. Communication signal 110 can be a wireless signal, such as Wi-Fi or Bluetooth, or a wired signal.

Base station 102 can comprise an interrogation device configured to transmit data to and receive data from tibial bearing 14, or another implant device, via communication signal 112. Communication signal 112 can be a wireless signal, such as Wi-Fi or Bluetooth. In additional examples, communication signal 112 can be configured to operate on one of more of the aforementioned IEEE 802.15.6-2012 protocol, an MICS protocol and an MBANs protocol.

Tibial bearing 14 can include electronics module 40 that can include an energy harvesting device. In examples, the energy harvesting device can comprise an RF energy harvester that can receive RF energy 114 from stray signals 116. Examples of energy harvesting devices that can be used in electronics module 40 are described in Pat. No. US 9,021,277 to Shearer et al. (Powercast) titled "Powering Devices Using RF Energy Harvesting," which is incorporated herein by this reference.

Base station 102 can be configured to generate wireless power-generating signal 118. Power-generating signal 118 can comprise an RF filed that can interact with the energy harvesting device of tibial bearing 14. The strength of power-generating signal 118 can be suitable to provide signal coverage of a home, dwelling or building such that a patient, e.g., a recipient of tibial bearing 14, can be freely wander around the home, dwelling or building within range of wireless power-generating signal 118. In examples, the strength and range of power-generating signal 118 can be commensurate with typical home Wi-Fi devices, such as routers and repeaters.

Implant monitoring system 100 can thus be used to communicate with and power tibial bearing 14 when a patient is within a predefined area where home base station 102 can be set-up and left to freely operate. However, when the patient needs to leave the predefined area, tibial bearing 14 can obtain power from RF energy 114, as can be present from stray RF signals and ambient RF signals. As such, the patient can be freed from having to frequently take specific actions to provide power to tibial bearing 14.

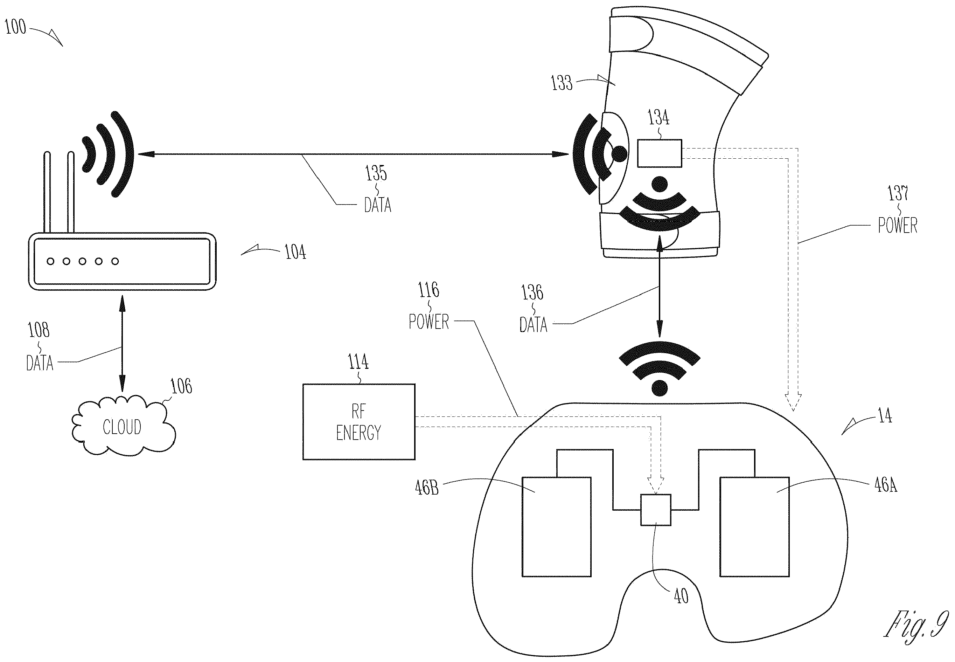

Data from tibial bearing 14 can be transmitted from home base station 102, to router 104 and to the cloud 106, whereby the data can be securely accessed by authorized personnel, such as caregivers, surgeons, rehabilitation therapists and the like. Thus, hospitals, home computers of the patient and other care providers can access the data from tibial bearing 14. In examples, base station 102 can be set up in a medical facility, such as a hospital, outpatient clinic or operating room. Thus, base station 102 can be used to obtain intraoperative data during the implantation procedure of tibial bearing 14, which is further described with reference to FIG. 9.

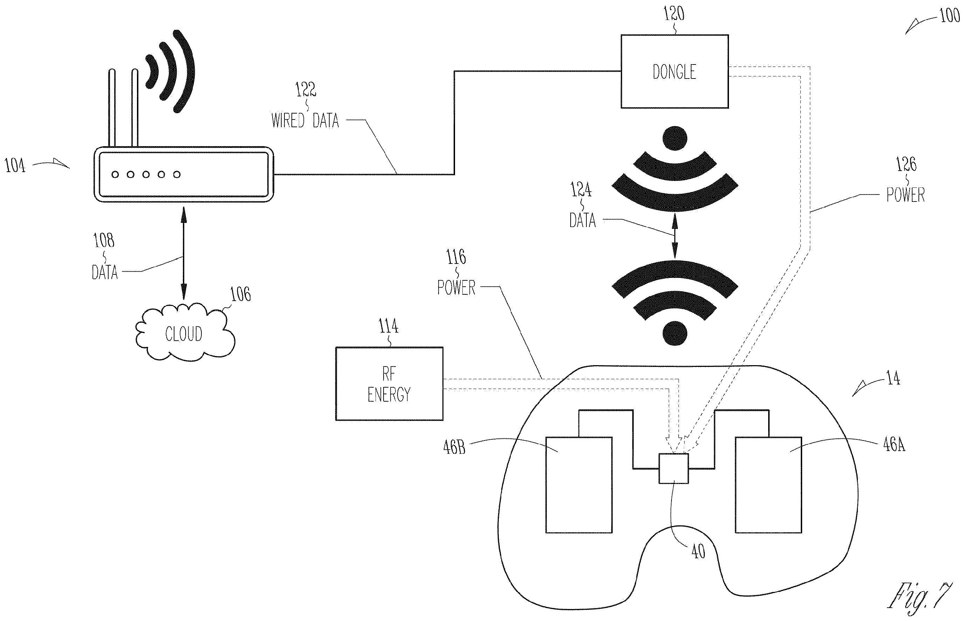

FIG. 7 is a schematic illustration of tibial bearing 14 of FIGS. 1-3 communicating with dongle 120 connected to wireless router 104 of FIG. 6. Wireless router 104 can be configured to communicate with the internet or the cloud 106 via signal 108. Wireless router 104 can be configured to communicate with dongle 120 via direct connection 122, which can comprise a wired connection.

Dongle 120 can comprise an interrogation device configured to transmit data to and receive data from tibial bearing 14, or another implant device, via communication signal 124. Communication signal 124 can be a wireless signal, such as Wi-Fi or Bluetooth. In additional examples, communication signal 124 can comprise one of more of the aforementioned IEEE 802.15.6-2012 signal, an MICS signal and an MBANs signal. Dongle 120 can be configured to generate wireless power-generating signal 126.

Tibial bearing 14 can include electronics module 40 that can include an energy harvesting device, as discussed herein. In examples, the energy harvesting device can comprise an RF energy harvester that can receive RF energy 114 from stray signals 116.

Dongle 120 can be configured to operate similarly as home base station 102. However, dongle 120 can be configured for simplified or streamlined operation. Thus, dongle 120 can be configured for plug-and-play functionality with already operating equipment, such as router 104, a desktop computer or a notebook computer. In examples, dongle 120 can comprise a Universal Serial Bus (USB) device that can be plugged into a corresponding port in router 104. Dongle 120 can be plugged into a laptop computer or desktop computer, or any other internet connected device having a USB connection. In additional examples, dongle 120 can comprise a mini- or micro-USB plug for connection to mobile phones and the like. For example, connection 122 can be used to connect to mobile computing device 84 (FIG. 5B). Thus, dongle 120 can be easily transported by a patient to different setting, e.g., home, workplace, vacation destinations, without having to set-up and configure a separately operating device. Likewise, dongle 120 can be easily set-up and used in a hospital or operating room.

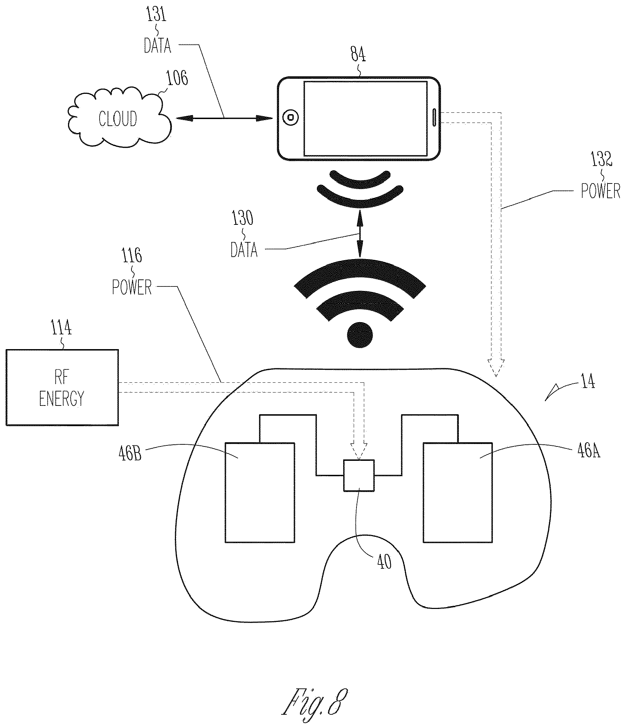

FIG. 8 is a schematic illustration of tibial bearing 14 of FIGS. 1 - 3 in direct communication with mobile computing device 84. Tibial bearing 14 and mobile computing device 84 can be configured to directly interact via communication signal 130 and power-generation signal 132. Mobile computing device 84 can further be in communication with the cloud 106 via communication signal 131 to exchange data.

Tibial bearing 14 and mobile computing device 84 can be configured to operate similarly as base station 102 and dongle 120, except that the ability to generate a power-generation signal, such as be inclusion of a RF power harvesting device described herein, can be incorporated directly into mobile computing device 84. Thus, mobile computing device 84 can provide access to the cloud 106, data communication with tibial bearing 14 and power capabilities to tibial bearing 14 without need of an intervening device.

FIG. 9 is a schematic illustration of tibial bearing 14 of FIGS. 1-3 communicating with wearable joint brace 133 that is in communication with wireless router 104. Wearable joint brace 133 can comprise electronics device 134 that can be configured to communicate with router 104 via signal 135 and tibial bearing 14 via signal 136. Electronics device 134 can provide power to tibial bearing 14 via wireless power-generating signal 137. In examples, wireless router 104 can be set-up and operated in an operating room where tibial bearing 14 is to be implanted into a patient.

Electronics device 134 can be configured as a wearable or portable module that can be easily transported and used in different environments. Electronics device 134 can include a power source such as rechargeable batteries. The power from the batteries within electronics device 134 can be replenished by conventional wireless and wired recharging methods. The power from the batteries within electronics device 134 can be transferred to tibial bearing 14 via wireless methods described herein, such as inductive charging or RF charging via energy harvesting.

Brace 133 can comprise a brace, sleeve or garment used to provide rehabilitation to a joint. Such braces can comprise compressive sleeves and sleeves reinforced with brackets to provide external stabilization to a joint. In examples, brace 133 can comprise a brace as is described in Pat. No. US 5,334,135 to Grim et al. titled "Formed Resilient Orthopaedic Support," which is incorporated herein by this reference. Brace 133 can include a pouch, pocket or sleeve into which electronics device 134 can be inserted. The pouch, pocket or sleeve can be located in position to facilitate interaction between tibial bearing 14 and electronics device 134 to exchange power and data.

In examples, electronics device 134 can be used with a brace or sleeve similar to brace 133 during the implantation procedure. The electronics device 134 can be powered by any of the methods described herein during the procedure, such as stray RF energy or energy provided by electronics device 134 via an appropriate power-transferring or power-generating signal. Thus, the surgeon can obtain appropriate data from the sensors, e.g., pressure data, to facilitate implantation of total knee prosthetic 10 (FIG. 1) in kinematic alignment. After the implantation procedure, the patient can be sent home with electronics device 134, after being cleaned and sterilized, as well as a new brace or sleeve similar to brace 133 for home use. The home-use brace or sleeve can be a rehabilitative brace having appropriate functionality to provide stability to the knee joint, or a simple sleeve to hold electronics device 134 against the patient. In examples, electronics device 134 can be configured as dongle 120 (FIG .7) with the addition of an independent power source for use without having to be connected to an external device such as router 104.

FIG. 10 is a block diagram illustrating components of electronics module 40 of tibial bearing 14. Although described with reference to tibial bearing 14, electronics module 40 can be configured for use with other orthopedic implant devices, such as prosthetic femoral components, prosthetic acetabular components, prosthetic humeral components and the like. Electronics module 40 can comprise housing 26, circuit board 140, processor 142, memory 144, switch 146, input/output (I/O) device 148, power source 150A, power source 150B, charging device 152, power harvesting device 154, communication device 156, first sensor 158A and second sensor 158B. Housing 26 can be attached to or integral with tibial bearing 14. Electronics module 40 can be in communication with interrogation device 160. Interrogation device 160 can comprise various electronics devices for powering electronics module 40 and obtaining information from electronics module 40, such as mobile computing device 84, base station 102, dongle 120 and electronics device 134. Though described with reference to tibial bearing 14, electronics module 40 can be used with other prosthetic implants.

Housing 26 can comprise a structural component to hold and support other components of electronics module 40. Housing 26 can be integral with, attached to, or disposed inside of tibial bearing 14. Housing 26 can be made of a medical grade plastic material, or can be made of other medical grade materials, such as stainless steel. Housing 26 can be made of a transparent or translucent material to facilitate transmission of light through housing 26 to improve visibility of any light sources disposed in or on housing 26. Housing 26 can be sealed to keep the components therein dry and away from engagement with the environment of electronics module 40.

Circuit board 140 can comprise a structural component for electrically and structurally coupling electrical components of electronics module 40. For example, circuit board 140 can comprise a silicon wafer or a chip onto which electrical couplings are attached for coupling switch 146, processor 142, memory 144, sensors 158A and 158B and the like.

Processor 142 can comprise an integrated circuit that controls operation of components of electronics module 40, such as I/O device 148, charging device 152, power harvesting device 154, communication device 156 and sensors 158A and 158B. Processor can execute instructions stored in memory 144 to operate components of electronics module 40, such as charging device 152, power harvesting device 154, and sensors 158A and 158B.

Memory 144 can comprise any suitable storage device, such as non-volatile memory, magnetic memory, flash memory, volatile memory, programmable read-only memory and the like. Memory 144 can include instructions stored therein for processor 142 to control operation of electronics module 40. For example, memory 144 can include instructions for operating I/O device 148, charging device 152, power harvesting device 154, communication device 156 and sensors 158A and 158B, as well as coordinating output from electronics module 40. Memory 144 can additionally include reference data for comparing data from sensors 158A and 158B, such as threshold conditions or pressures for when femoral component 16 and tibial component 12 (FIG. 1) are in kinematic alignment or when rechargeable power source 150B is charged or not charged.

Switch 146 can comprise a an on/off switch for providing power from power sources 150A and 150B to sensors 158A and 158B, etc. Switch 146 can comprise an "alternate action" switch when transitioning between open or closed states. In alternate action switches, a switch can be flipped for continuous "on" or "off' operation. Switch 146 can comprise a toggle switch, a knife switch, a relay or a push-button switch. In examples, electronics module 40 does not include a switch and electronics module 40 can be powered on so long as one of primary power source 150A and rechargeable power source 150B is at least partially charged.

I/O device 148 can comprise one or more devices for receiving input from and sending output to a user of electronics module 40. In order to operate or obtain information from electronics module 40. I/O device 148 can comprise a button, a knob, a dial and the like. In examples, I/O device 148 can be omitted and electronics module 40 can communicate with interrogation device 160 in order to operate electronics module 40.

I/O device 148 can comprise devices for providing visual and audio feedback. I/O device 148 can comprise a device for producing light waves, such as incandescent light bulbs, light-emitting-diodes and the like. In examples, I/O device 148 can be configured for emitting different colors or wavelengths of light. I/O device 148 can provide visual indications of when electronics module 40 is performing different functions, such as actively sensing. For example, I/O device 148 can be configured to emit orange, yellow and green light, so that an operator can confirm that different functions of electronics module 40 are being performed, or that a loss of communication or a malfunction of electronics module 40 is occurring.

I/O device 148 can include or comprise a device for making waves, such as a sound wave or a vibration wave. In an example, I/O device 148 can comprise an auditory device, such as a speaker or amplifier for producing an auditory signal or sound to indicate that electronics module 40 is in communication with interrogation device 160. In other examples, I/O device 148 can comprise tactile device, such as a reciprocating or oscillating device, for producing a vibration that can be felt by a surgeon, operator of interrogation device 160 or patient. For example, a wave can communicate with a device worn by a surgeon at interrogation device 160 that can vibrate when receiving the wave.

Communication device 156 can comprise one or more devices for receiving input from interrogation device 160 or providing an output to interrogation device 160 via various signals. Communication device 156 can provide signal 162 to interrogation device 160. Interrogation device 160 can thereafter, for example, display on human interface device 164, such as a video display monitor, an indication of information from electronics module 40. Interrogation device 160 can further comprise I/O device 166 to receiving input from and send output to a user of interrogation device 160, such as a surgeon.

Communication device 156 can receive signal 162 from interrogation device 160 for storing information on memory 144 or providing information to processor 142 for operating switch 146, charging device 152, power harvesting device 154, sensors 158A and 158B, communication device 156, charging device 152 and energy harvesting device 154 and other components of electronics module 40. In examples, communication device 156 can communicate using wireless communications signals, such as Bluetooth, WiFi, Zigbee, infrared (IR), near field communication (NFC), 3GPP or other technologies. In examples, communication device 156 can comprise a wired connection or can include a port for receiving a wire for a wired connection. In examples, communication device 156 can communicate using one of more of the aforementioned IEEE 802.15.6-2012 protocol, an MICS protocol and an MBANs protocol.

Communication device 156 can optionally be used in conjunction with antenna relay 180 that extends outside of housing 26. Antenna relay 180 can comprise an independently implantable component that can be located within tissue between electronics module 40 and the skin. Antenna relay 180 can be uncoupled from each of electronics module 40 and the orthopedic implant. As such, antenna relay 180 can comprise an intermediary to allow native communication capabilities of communication device 156 to be enhanced or relayed outside of the patient with a stronger signal. Antenna relay 180 can be energized with power from interrogation device 160 to receive and rebroadcast a signal from electronics module 40.

Power source 150A can comprise an energy storage device such as a battery including an electrochemical cell, such as an alkaline or zinc-manganese battery. In examples, power source 150A can comprise a primary, or non-rechargeable battery.

Power source 150B can comprise a rechargeable battery. Power source 150B can comprise lead 168. Lead 168 can provide a conductor for charging power source 152B. Power source 150B can additionally be configured for contactless charging, such as via induction charging.

In examples, one or both of power sources 150A and 150B can comprise one or more capacitors that can be charged by charging device 152, such as via an inductance signal, a magnetic signal or an RF energy signal. Thus, the capacitors can be charged by, for example, operation of an RF energy harvester, to provide a suitable amount of power to steadily operate electronics module 40 while the RF energy being harvested may fluctuate due to varying RF energy signal strength.

Power sources 150A and 150B can be configured to provide power to different components of electronics module 40. Primary battery, or power source 150A can provide long term battery power and can provide power to low-frequency sensor operations over the lifetime of electronics module 40. Rechargeable battery, or power source 150B can provide short term battery power and can provide short duration, high frequency sensor operations, such as during exercise conducted as part of post-operative rehabilitation. Electronics module 40 can include one or more of each of power sources 150A and 150B. Furthermore, in examples, electronics module 40 can exclude both of power sources 150A and 150B and can rely solely on power generated by energy harvesting device 154. In examples, power sources 150A can comprise a capacitor and power source 150B can comprise an emergency or back-up power source, such as a non-rechargeable battery.

Charging device 152 can comprise one or more devices for providing power to power sources 150A and 150B. In examples, charging device 152 can comprise a coil that can be energized by an RF field. In examples, charging device 152 can comprise a coil that can be energized by a magnetic field. In examples, charging device 152 can comprise a magnetic loop antenna, such as a copper coil. In examples, charging device 152 can be energized by wireless power-sharing signal 94.

Energy harvesting device 154 can comprise one or more devices for providing power to power sources 152A and 152B. Energy harvesting device 154 can comprise a device for converting radio frequency energy or waves into electricity. Examples of energy harvesting device 154 are described in Pat. No. US 9,021,277 to Shearer et al. (Powercast) titled "Powering Devices Using RF Energy Harvesting," which is incorporated herein by this reference.

Sensors 158A and 158B can comprise a variety of different sensors, such as temperature, pH, force, vibration, impact, position, motion, capacitance, conductance, impedance and the like. Only one of sensors 158A and 158B can be included in electronics module 40 or more than two sensors can be included in electronics module 40. Sensors 158A and 158B can comprise sensors 46, 46A and 46B, as described with reference to FIGS. 2 and 3. Sensors 158A and 158B can include leads 174 that can provide for remote sensing capabilities outside of housing 26. Leads 174 can comprise cables that are a bundle of other cables or wires relating to, among other things, power transmission, data transmission and signal transmission.

Electronics module 40 can be customized to include only the sensors and input and output devices that are desired for a particular procedure.

The systems, devices and methods discussed in the present application can be useful in providing implantable medical devices that have sensors to collect patient and implant data. The implantable medical devices can be appropriately sized and shaped without having to incorporate bulky batteries or can include smaller batteries because, for example, the implantable medical devices can be powered using wireless power signals. The wireless powering signals can comprise inductance charging signals from a mobile phone. The wireless powering signals can be radio frequency signals from RF generators or ambient or stray radio frequency signals in the environment. The radio frequency signals can be used to power RF energy harvesters within the implantable medical devices that can be activated to power the sensors, communication signal generators and the like. RF generators can be set up in areas of operation of the patient, such as the home and workplace. The RF generators can be power the implantable medical devices with strong and consistent radio frequency signals in environments where the patient frequents. When the patient is outside these environments, ambient radio frequency signals can be used to provide sufficient power to keep the implantable medical device operating for short periods or operating in short intervals until the patient and the implantable medical device return to an environment where an RF generator is located. As such, the patient need not worry about repeatedly connecting the implantable medical device to a charging station or the like. Furthermore, the RF generators can be used intraoperatively while the implantable medical device is being implanted to provide feedback to the surgeon to facilitate proper implantation of the device.

### Examples

Example 1 is a surgical sensor system for collecting internal patient data, the surgical sensor system comprising: a prosthetic implant comprising: a housing; a sensor disposed within the housing; and an internal power device connected to the sensor; and an external interrogation device comprising: a wireless power signal generator for activating with the internal power device of the prosthetic implant.

In Example 2, the subject matter of Example 1 optionally includes wherein: the prosthetic implant comprises a wireless communication device; and the external interrogation device comprises a wireless communication signal generator for exchanging data with the wireless communication device.

In Example 3, the subject matter of Example 2 optionally includes the wireless communication device being configured to communicate using an IEEE 802.15.6-2012 protocol, a Medical Implant Communication Service (MICS) protocol, or a Medical Body Area Networks (MBANs) protocol.

In Example 4, the subject matter of any one or more of Examples 2-3 optionally include wherein the external interrogation device further comprises a communications interface for the Internet.

In Example 5, the subject matter of any one or more of Examples 1-4 optionally include wherein: the internal power device comprises a radio frequency energy harvester; and the wireless power signal generator comprises a radio frequency signal generator.

In Example 6, the subject matter of Example 5 optionally includes wherein the internal power device comprises a capacitor to store energy generated by the radio frequency energy harvester.

In Example 7, the subject matter of any one or more of Examples 1-6 optionally include wherein: the internal power device comprises a battery; and the wireless power signal generator comprises a wireless charging signal generator.

In Example 8, the subject matter of any one or more of Examples 1-7 optionally include wherein: the external interrogation device comprises a mobile phone; and the prosthetic implant comprises an artificial hip component.

In Example 9, the subject matter of any one or more of Examples 1-8 optionally include wherein the sensor comprises at least one of a pressure sensor, an accelerometer and a gyroscope.

In Example 10, the subject matter of Example 9 optionally includes wherein the prosthetic implant comprises a tibial bearing.

In Example 11, the subject matter of any one or more of Examples 1-10 optionally include wherein: the internal power device comprises a rechargeable battery; the external interrogation device comprises a mobile phone; and the wireless power signal generator is configured to generate a wireless power-sharing signal.

In Example 12, the subject matter of any one or more of Examples 1-11 optionally include wherein: the internal power device comprises a radio frequency energy harvester; the external interrogation device comprises a base station; and the wireless power signal generator is configured to generate a radio frequency signal.

In Example 13, the subject matter of any one or more of Examples 1-12 optionally include wherein: the internal power device comprises a radio frequency energy harvester; the external interrogation device comprises a dongle configured to plug into a wireless router; and the wireless power signal generator is configured to generate a radio frequency signal.

In Example 14, the subject matter of any one or more of Examples 1-13 optionally include wherein: the internal power device comprises a radio frequency energy harvester; the external interrogation device comprises a mobile phone; and the wireless power signal generator is configured to generate a radio frequency signal.

In Example 15, the subject matter of any one or more of Examples 1-14 optionally include wherein: the internal power device comprises a radio frequency energy harvester; the external interrogation device comprises a portable module comprising a rechargeable battery; and the wireless power signal generator is configured to generate a radio frequency signal.

In Example 16, the subject matter of Example 15 optionally includes wherein the portable module is configured to communicate with the Internet.

In Example 17, the subject matter of any one or more of Examples 15-16 optionally include a joint brace device configured to carry the portable module.

Example 18 is a medical implant sensor interrogation device comprising: a communication interface configured to communicate with the Internet via a wireless signal; a radio frequency signal generator configured to activate a radio frequency energy harvester; and a sensor communication device configured to generate a low-power, human anatomy compatible communication signal.

In Example 19, the subject matter of Example 18 optionally includes the sensor communication device being configured to generate an IEEE 802.15.6-2012 protocol compatible signal.

In Example 20, the subject matter of any one or more of Examples 18-19 optionally include a joint brace device configured to carry the medical implant sensor interrogation device.

Example 21 is a method of remotely interacting with a sensor device implanted in anatomy with an orthopedic device, the method comprising: generating a wireless powering signal; activating the sensor device with the wireless power signal; collecting sensor data from the sensor device; and wirelessly communicating the sensor data from the sensor device using a low-power wireless signal.

In Example 22, the subject matter of Example 21 optionally includes the low-power wireless signal generating an IEEE 802.15.6-2012 compliant signal.

In Example 23, the subject matter of any one or more of Examples 21-22 optionally include wherein the low-power wireless signal comprises generating a signal compliant with a Medical Implant Communication Service (MICS) protocol or a Medical Body Area Networks (MBANs) protocol.

In Example 24, the subject matter of any one or more of Examples 21-23 optionally include wherein generating the wireless powering signal comprises generating an inductive charging signal.

In Example 25, the subject matter of Example 24 optionally includes the wireless powering signal is generated using a mobile phone.

In Example 26, the subject matter of any one or more of Examples 24-25 optionally include attaching the mobile phone to the patient at an anatomic location of the sensor device implanted in the anatomy.

In Example 27, the subject matter of any one or more of Examples 21-26 optionally include wherein generating the wireless powering signal comprises generating a radio frequency signal.

In Example 28, the subject matter of Example 27 optionally includes the wireless powering signal is generated using a portable module.

In Example 29, the subject matter of any one or more of Examples 27-28 optionally include the wireless powering signal is generated using a base station.

In Example 30, the subject matter of any one or more of Examples 27-29 optionally include wherein activating the sensor device with the wireless power signal comprises generating power with an RF energy harvester.

In Example 31, the subject matter of Example 30 optionally includes power in a capacitor electrically connected to the RF energy harvester.

In Example 32, the subject matter of any one or more of Examples 30-31 optionally include activating the sensor device with ambient RF energy.

In Example 33, the subject matter of any one or more of Examples 21-32 optionally include intraoperatively collecting the sensor data.

In Example 34, the subject matter of any one or more of Examples 21-33 optionally include attaching the portable module to the anatomy with a garment.

Example 35 is a method of intraoperatively receiving sensor data from an implantable sensor-enabled orthopedic device, the method comprising: generating a wireless powering signal within an operating room using an interrogation device; activating electronics within the orthopedic device with the wireless power signal; collecting sensor data from the electronics; and wirelessly communicating sensor data from the electronics to the interrogation device.

In Example 36, the subject matter of Example 35 optionally includes implanting the orthopedic device into anatomy of the patient.

In Example 37, the subject matter of Example 36 optionally includes adjusting kinematic alignment of the orthopedic device intraoperatively based on the communicated sensor data.

In Example 38, the subject matter of any one or more of Examples 35-37 optionally include wherein the interrogation device comprises a portable module comprising: self-powering capability; and a communication signal generator configured to generate a communication signal that is IEEE 802.15.6-2012 compliant.

In Example 39, the subject matter of Example 38 optionally includes attaching the interrogation device to an exterior of the patient using a garment or brace.

In Example 40, the subject matter of any one or more of Examples 35-39 optionally include wherein activating electronics within the orthopedic device with the wireless power signal comprises generating power with an RF energy harvester.

Each of these non-limiting examples can stand on its own, or can be combined in various permutations or combinations with one or more of the other examples.

### Various Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided. Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.
The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A surgical sensor system for collecting internal patient data, the surgical sensor system comprising:
a prosthetic implant comprising:
a housing;
a sensor disposed within the housing; and
an internal power device connected to the sensor; and
an external interrogation device comprising:
a wireless power signal generator for activating the internal power device of the
prosthetic implant.

2. The surgical sensor system of claim 1, wherein:
the prosthetic implant comprises a wireless communication device; and
the external interrogation device comprises a wireless communication signal generator for exchanging data with the wireless communication device.

3. The surgical sensor system of claim 2, wherein the wireless communication device is configured to communicate using an IEEE 802.15.6-2012 protocol, a Medical Implant Communication Service (MICS) protocol, or a Medical Body Area Networks (MBANs) protocol.

4. The surgical sensor system of claim 2, wherein the external interrogation device further comprises a communications interface for the Internet.

5. The surgical sensor system of claim 1, wherein:
the internal power device comprises a radio frequency energy harvester; and
the wireless power signal generator comprises a radio frequency signal generator
wherein optionally the internal power device comprises a capacitor to store energy generated by the radio frequency energy harvester.

6. The surgical sensor system of claim 1, wherein:
the internal power device comprises a battery; and
the wireless power signal generator comprises a wireless charging signal generator.

7. The surgical sensor system of claim 1, wherein:
the external interrogation device comprises a mobile phone; and
the prosthetic implant comprises an artificial hip component or the prosthetic implant comprises a tibial bearing.

8. The surgical sensor system of claim 1, wherein the sensor comprises at least one of a pressure sensor, an accelerometer and a gyroscope.

9. The surgical sensor system of claim 1, wherein:
the internal power device comprises a rechargeable battery;
the external interrogation device comprises a mobile phone; and
the wireless power signal generator is configured to generate a wireless power-sharing signal.

10. The surgical sensor system of claim 1, wherein:
the internal power device comprises a radio frequency energy harvester;
the external interrogation device comprises a base station; and
the wireless power signal generator is configured to generate a radio frequency signal.

11. The surgical sensor system of claim 1, wherein:
the internal power device comprises a radio frequency energy harvester;
the external interrogation device comprises a dongle configured to plug into a wireless router or the external interrogation device comprises a mobile phone or the external interrogation device comprises a portable module comprising a rechargeable battery; and
the wireless power signal generator is configured to generate a radio frequency signal.

12. The surgical sensor system of claim 11, wherein the portable module is configured to communicate with the Internet.

13. The surgical sensor system of claim 11, further comprising a joint brace device configured to carry the portable module.

14. A method of remotely interacting with a sensor device previously implanted in anatomy with an orthopedic device, the method comprising:
generating a wireless powering signal;
activating the sensor device with the wireless power signal;
collecting sensor data from the sensor device; and
wirelessly communicating the sensor data from the sensor device using a low-power wireless signal.

15. The method of claim 14, wherein the low-power wireless signal comprises generating an IEEE 802.15.6-2012 compliant signal and optionally wherein the low-power wireless signal comprises generating a signal compliant with a Medical Implant Communication Service (MICS) protocol or a Medical Body Area Networks (MBANs) protocol.
